# EUROPEAN PATENT APPLICATION

(11) **EP 1 424 070 A1**
(43) Date of publication of application: **02.06.2004**
(21) Application number: 02292940.0
(22) Date of filing: 28.11.2002
(51) Int. Cl.: A61K 31/192, A61K 31/216, A61K 31/195, A61P 3/00, A61K 31/155

(54) **Combination of a PPAR alpha agonist and metformin for decreasing the serum triglycerides**

(71) Applicant: Fournier Laboratories Ireland Limited, Carrigtwohill, Co. Cork (IE)
(72) Inventor: Junien, Jean-Louis, 92310 Sevres (FR); Edgar, Alan, 21490 Saint-Julien (FR); Chaput, Evelyne, 21000 Dijon (FR)
(74) Representative: Nevant, Marc

(57) **Abstract**

The present invention relates to the combined use of a PPARα agonist and metformin for decreasing serum triglycerides.

## Description

The present invention relates to the use of a PPARα agonist and metformin for decreasing serum triglycerides.

Metabolic Syndrome also called Syndrome X is the syndrome characterised by an initial insulin resistant state, generating hyperinsulinaemia, dyslipidaemia and impaired glucose tolerance, which can progress to non-insulin dependent diabetes mellitus (Type II diabetes), characterised by hyperglycaemia and which then further progresses to diabetic complications.

The third set of guidelines issued in May 2002 by the National Cholesterol Education Program (NCEP) differs from the second set issued in 1993 in several ways. Although reducing elevated levels of low-density lipoprotein (LDL) cholesterol remains the primary focus of therapy, the new NCEP guidelines also include strategies to identify and treat patients with low levels of high-density lipoprotein (HDL) cholesterol and/or elevated triglyceride levels. Just as there is "good" cholesterol (HDL) and "bad" cholesterol (LDL), there are also "good" triglyceride-containing lipoproteins, which contain high concentrations of triglyceride remnants and are associated with low risk, and "bad" triglyceride-containing lipoproteins, which contain high concentrations of cholesterol remnants and are associated with increased risk. The mechanisms by which "bad" triglycerides develop explain why elevated triglycerides and low HDL, and patients with the metabolic syndrome, warrant special attention. These mechanisms and others also suggest new targets for therapeutic intervention and the development of new drugs that will correct lipid and lipoprotein abnormalities through a number of different metabolic pathways.

According to these guidelines, clinical identification of the Metabolic Syndrome appears with any 3 of the Following:

| **Risk Factor** | **Defining Level** |
|---|---|
| Abdominal obesity | Waist circumference |
| Men | > 102 cm |
| Women | >88 cm |
| Triglycerides HDL cholesterol | > 150 mg/dL |
| Men | <40 mg/dl |
| Women | <50 mg/dl |
| Blood pressure | >130/ 85 mmHg |
| Fasting glucose | >110 mg/dL |

The treatment of the metabolic syndrome suggested in the guidelines focuses on the treatment of the underlying causes (overweight/obesity and physical inactivity) by intensifying weight management and by increasing physical activity.

The treatment of lipid and non-lipid risk factors, if they persist despite these lifestyle therapies, is advised by treating hypertension, using aspirin for CHD patients to reduce their prothrombotic state and treating elevated triglycerides and/or low HDL.

The use of PPARα agonists is known for the treatment of elevated triglycerides.

PPARα is a subtype of the PPAR (Peroxisome Proliferator Activated Receptor) family. PPARα is predominantly expressed in tissues catabolizing high amounts of fatty acids, such as liver, heart and brown adipose tissue. Activated PPARs form heterodimers with RXR (Retinoid X Receptor) and the heterodimer binds to a specific response element, termed PPRE (PPAR Response Element), in the regulatory regions of target genes and subsequently alters their transcription. The majority of the genes whose expression is under control of PPARα code for proteins involved in intra- and extracellular lipid metabolism, such as acyl coA oxidase, acyl-coA synthetase and apolipoproteins A-I, A-II and C-III.

Fibrates can be cited as PPARα activators or agonists. In the present invention, the term agonist or activator is used equally to designate a compound that can activate a PPAR receptor.

Fibrates have been documented to lower plasma triglycerides and cholesterol levels and to be beneficial in the prevention of ischemic heart disease in individuals with dyslipidemia. They can also modestly decrease elevated fibrinogen and PAI-1 levels. Fibrate compounds, e.g., gemfibrozil, fenofibrate, bezafibrate, and ciprofibrate, elevate the level of plasma HDL cholesterol.

Metformin is mainly known for its anti-hyperglycaemic activity and is widely used in the treatment of non-insulin-dependent diabetes. In the case of insulin-dependent diabetes, metformin is also administered to the patient in combination with insulin.

EP 1054665 discloses a combination of metformin and of a fibrate chosen from fenofibrate and bezafibrate for the treatment of non-insulin-dependent diabetes. The synergistic effect observed lies in a marked improvement of the hypoglycaemia.

Surprisingly, the present inventors have now discovered that a combination of metformin with a PPARα activator leads to a significant improvement of the treatment of patients with elevated triglycerides. More specifically, a synergistic effect has been obtained by the combined administration of metformin and a PPARα agonist. The synergistic effect observed lies in a marked improvement of the level of triglycerides.

It has thus unexpectedly been found that a combination of a PPARα agonist with metformin presents an enhanced therapeutic potential in the metabolic syndrome aetiology due to an enhanced hypolipidaemic effect.

It therefore is an objective of the present invention to provide the use of a PPARα agonist and metformin for decreasing the serum triglycerides.

A further object of the present invention is the use of a PPARα agonist, metformin and a pharmaceutically acceptable carrier for the manufacture of a pharmaceutical formulation for decreasing serum triglycerides.

As explained above, elevated triglycerides level is related to the metabolic syndrome. Hypertriglyceridemia is also involved in the development of the metabolic syndrome as elevated levels of serum triacylglycerols impair tissue utilization of glucose.

Thus, the present invention is also directed to the use of a PPARα agonist and metformin for the treatment of metabolic syndrome.

The present invention is further directed to the use of a PPARα agonist, metformin and a pharmaceutically acceptable carrier for the manufacture of a pharmaceutical formulation for the treatment of metabolic syndrome.

Hypertriglyceridemia also leads to the accumulation of triacylglycerol in adipose tissue and hence the development of obesity.

Thus, the present invention is also directed to the use of a PPARα agonist and metformin for the treatment of obesity.

The present invention is further directed to the use of a PPARα agonist, metformin and a pharmaceutically acceptable carrier for the manufacture of a pharmaceutical formulation for the treatment of obesity.

By "PPARα agonist" is meant a compound or composition which when combined with PPARα directly or indirectly (preferably binding directly to PPARα) stimulates or increases an *in vivo* or *in vitro* reaction typical for the receptor, e.g. transcriptional regulation activity, as measured by an assay known to one skilled in the art, including, but not limited to, the "co-transfection" or "cistrans" assays described or disclosed in U.S. Patent Nos. 4,981,784, 5,071,773, 5,298,429, 5,506,102, WO89/05355, WO91/06677, WO92/05447, WO93/11235, WO93/23431, WO94/23068, WO95/18380, CA 2,034,220, and Lehmann, et al., J. Biol. Chem. 270:12953-12956 (1995), which are incorporated by reference herein. PPARα agonists may also be identified according to an assay described in US Patent 6,008,239.

A preferred PPARα agonist is a fibrate compound including, but not limited to, gemfibrozil, fenofibrate, bezafibrate, clofibrate, ciprofibrate, and analogues, derivatives and pharmaceutically acceptable salts thereof. PPARα compounds disclosed in Tontonez et al., Cell 79:1147-1156 (1994), Lehmann et al., J. Biol. Chem. 270(22):1-4, 1995, Amri et al., J. Lipid Res. 32:1449-1456 (1991), Kliewer et al., Proc. Natl. Acad. Sci. USA 94:4318-4323 (1997), Amri et al., J. Lipid Res. 32:1457-1463, (1991) and Grimaldi et al., Proc. Natl. Acad. Sci. USA 89:10930-10934 (1992) are incorporated by reference herein. PPARα agonist compounds described in US Patent 6,008,239, WO 97/27847, WO 97/27857, WO 97/28115, WO 97/28137 and WO 97/28149 are further incorporated by reference herein. Certain fibrate compounds as described in WO92/10468 and WO01/80852 are also incorporated by reference herein.

In the present invention, fibrates include fibric acid derivatives and pharmaceutically acceptable salts and esters of such fibric acid derivatives. Fibric acid derivatives lower the levels of triglyceride-rich lipoproteins, such as VLDL, raise HDL levels, and have variable effects on LDL levels. The effects on VLDL levels appear to result primarily from an increase in lipoprotein lipase activity, especially in muscle. This leads to enhanced hydrolysis of VLDL triglyceride content and an enhanced VLDL catabolism. Fibric acid agents also may alter the composition of the VLDL, for example, by decreasing hepatic production of apoC-III, an inhibitor of lipoprotein lipase activity. These compounds are also reported to decrease hepatic VLDL triglyceride synthesis, possibly by inhibiting fatty acid synthesis and by promoting fatty acid oxidation.

Fenofibrate is commercially available as TricorTM capsules. Each capsule contains 67 mg of micronized fenofibrate.

Clofibrate is commercially available as Atromid-S capsules. Each capsule contains 500 mg of clofibrate. Clofibrate lowers elevated serum lipids by reducing the very low-density lipoprotein fraction rich in triglycerides. Serum cholesterol may be decreased. It may inhibit the hepatic release of lipoproteins (particularly VLDL) and potentiate the action of lipoprotein lipase. The recommended daily dose of clofibrate is 2 g, administered in divided doses.

Gemfibrozil is commercially available as Lopid tablets. Each tablet contains 600 mg of gemfibrozil. Gemfibrozil is a lipid regulating agent that decreases serum triglycerides and very low density lipoprotein cholesterol, and increases high density lipoprotein cholesterol. The recommended daily dose of gemfibrozil is 1200 mg, administered in two divided doses.

In the invention, the PPARα agonist can be a fibrate selected from the group consisting of gemfibrozil, fenofibrate, bezafibrate, clofibrate, ciprofibrate.

According to the present invention, the preferred fibrate is fenofibrate.

According to the invention, metformin can be administered in the form of one of its pharmaceutically acceptable salts, such as the hydrochloride, acetate, benzoate, citrate, fumarate, embonate, chlorophenoxyacetate, glycolate, palmoate, aspartate, methanesulphonate, maleate, parachlorophenoxylsobutyrate, formate, lactate, succinate, sulphate, tartrate, cyclohexanecarboxylate, hexanoate, octonoate, decanoate, hexadecanoate, octodecanoate, benzenesulphonate, trimethoxybenzoate, paratoluenesulphonate, adamantanecarboxylate, glycoxylate, glutamate, pyrrolidonecarboxylate, naphthalenesulphonate, 1-glucosephosphate, nitrate, sulphite, dithionate or phosphate.

Among these salts, the hydrochloride, fumarate, embonate and chlorophenoxyacetate are more particularly preferred.

The pharmaceutically acceptable salts of metformin are obtained in a manner which is known per se by the action of metformin on the corresponding acid.

When used herein "Metabolic Syndrome" includes the syndrome as defined in the third set of guidelines issued by the NCEP, i.e. Metabolic Syndrome appears with anv 3 of the following:

| **Risk Factor** | **Defining Level** |
|---|---|
| Abdominal obesity | Waist circumference |
| Men | > 102 cm |
| Women | >88 cm |
| Triglycerides | > 150 mg/dL |
| HDL cholesterol | |
| Men | <40 mg/dl |
| Women | <50 mg/dl |
| Blood pressure | >130/ 85 mmHg |
| Fasting glucose | >110 mg/dL |

For the avoidance of doubt, the use, methods and treatments of this invention encompass the prevention, treatment and/or prophylaxis of the metabolic syndrome.

In another embodiment, the invention includes a method of decreasing serum triglycerides, of treating the metabolic syndrome or of treating obesity comprising co-administering an effective dosage of a PPARα agonist and metformin, where the effective dosage of the PPARα agonist is in the range of about 10 to about 3000 mg per day, preferably in the range of about 50 to about 300 mg per day.

In a further embodiment, the invention includes a method for decreasing serum triglycerides, of treating the metabolic syndrome or of treating obesity comprising co-administering an effective dosage of a PPARα agonist and metformin, where the effective dosage of metformin is in the range of about 10 to about 3000 mg per day, preferably in the range of about 100 to about 1000 mg per day.

According to an embodiment of the invention, the amount of metformin or of its salt which is used is from one to twenty times the mass of the PPARα agonist, preferably from one to five times and better from two to five times.

In another embodiment, the PPARα agonist and the metformin are administered simultaneously or co-administered.

In another embodiment, the PPARα agonist and the metformin are administered sequentially.

As used in this application, "co-administration" means the administration of two or more compounds to the same patient, within a time period of up to about two to about twelve hours. For example, co-administration encompasses (1) simultaneous administration of a first and second compound; (2) administration of a first compound, followed by administration of a second compound about 2 hours after administration of the first compound; and (3) administration of a first compound, followed by administration of a second compound about 12 hours after administration of the first compound. As described herein, the present invention encompasses co-administration of a PPARα agonist and metformin to a patient.

According to the present invention, a pharmaceutical formulation is defined as the formulation of the active compound with encapsulating material as a carrier providing a capsule in which the active component with or without other carriers, is surrounded by a carrier, which is thus in association with it. This includes tablets, powders, capsules, pills, cachets, and lozenges which can be used as solid dosage forms suitable for oral administration.

An effective dosage is defined in the present invention as the amount of a compound that prevents or ameliorates adverse conditions or symptoms of disease(s) or disorder(s) being treated. With respect to the PPARα agonist and metformin, effective dosage means a pharmacological dose in the range defined above. With respect to fibrates, the skilled artisan will understand and appreciate that the effective dosage of a given fibrate will vary with the potency of the fibrate.

Pharmaceutical formulations of the PPARα agonist and/or metformin can be prepared according to known methods. The preferred route of administering the PPARα agonist and metformin is mucosal administration, most preferably oral administration.

For preparing pharmaceutical compositions containing a PPARα agonist and/or metFormin, pharmaceutically acceptable carriers can be either solid or liquid. Solid form preparations include powders, tablets, pills, capsules, cachets, suppositories, and dispersible granules. A solid carrier can be one or more substances which may also act as diluents, flavoring agents, binders, preservatives, tablet disintegrating agents, or an encapsulating material.

In powders, the carrier is a finely divided solid which is in admixture with the finely divided active component. In tablets, the active component is mixed with the carrier having the necessary binding properties in suitable proportions and compacted in the shape and size desired. The powders and tablets preferably contain from five or ten to about seventy percent of the active compound. Suitable carriers are magnesium carbonate, magnesium stearate, talc, sugar, pectin, dextrin, starch, gelatin, tragacanth, methylcellulose, sodium carboxymethylcellulose, a low melting wax, cocoa butter, and the like.

Liquid form preparations include solutions, suspensions, and emulsions, for example, water or water propylene glycol solutions. For parenteral injection liquid preparations can be formulated in solution e.g. in aqueous polyethylene glycol solution.

Aqueous solutions suitable for oral use can be prepared by dissolving the active component in water and adding suitable colorants, flavors, stabilizing and thickening agents as desired. Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

The pharmaceutical preparation of the invention can also be a kit comprising two separate compositions, the first comprising the PPARα agonist and the second comprising metformin or a pharmaceutically acceptable salt thereof.

As shown in the examples, the applicant unexpectedly found that a PPARα agonist and metformin significantly reduce the triglycerides level and thus that such a combination can be used for the treatment of the metabolic syndrome or for the treatment of obesity.

The invention is further illustrated by the following examples, which are not to be construed as limiting, but merely as an illustration of some preferred features of the invention.

### EXAMPLE 1 : Effect of PPARα agonist and metformin co-administration on triglycerides in ob/ob mice.

Studies were designed to evaluate the effects of fenofibrate, a PPARα agonist, and metformin as a combination therapy, on triglycerides level in ob/ob mice.

### METHOD

Male homozygous C57BL/Ks/Ola/Hsd/lep ob/ob mice were stabilized for 2 weeks in the animal facilities in a temperature-, humidity- and light- controlled room (21-23°C, 12-12h light-dark cycle). They were fed with a standard laboratory diet and had free access to water. After acclimatization, they were randomized into groups of 10 based on body weight, as follows:
Vehicle = untreated mice
Met 100 = mice treated once a day with metformin, 100 mg/kg
Feno 100 = mice treated once a day with fenofibrate, 100 mg/kg
Feno100-Met100 = mice treated once a day with fenofibrate, 100 mg/kg and metformin, 100 mg/kg.

Serum triglycerides (expressed in g/I) were measured at the beginning and the end of the study for each group.

The results are summarized in Table 1.

**Table 1:**

| results after 10 days of treatment | | |
|---|---|---|
| | T0 | T0+10 |
| Vehicle | 0.74 ± 0.04 | 0.79 ± 0.04 |
| Met 100 | 0.68 ± 0.03 | 0.74 ± 0.04 |
| Feno 100 | 0.72 ± 0.06 | 0.69 ± 0.03 |
| Feno 100 - Met 100 | 0.69 ± 0.02 | 0.55 ± 0.02 |
| Values are expressed as mean ± sem | | |

*Statistics:* All data were subjected to covariance analysis followed by Tukey test. The comparison between Fenofibrate 100 mg/kg versus Fenofibrate 100 mg/kg + Metformin 100 mg/kg was considered significant : p=0.0022.
The comparison between Metformin 100 mg/kg versus Fenofibrate 100 mg/kg + Metformin 100 mg/kg was considered significant : p=0.0002.

The data showed that the triglycerides level was decreased in a synergistic way when a PPARα agonist was administered in conjunction with metformin. It was unexpected that the decrease of the triglycerides level with the PPARα agonist treatment was further enhanced when metformin was combined to said PPARα agonist.

This is further unexpected as the metformin treatment showed no effects on the triglycerides.

### EXAMPLE 2 : Effect of PPARα agonist and metformin co-administration on triglycerides in db/db mice.

Studies were designed to evaluate the effects of fenofibrate, a PPARα agonist, and metformin as a combination therapy, on triglycerides level in db/db mice.

### METHOD

Male 11/12 weeks old C587BL/Ks J Rj-db (db/db) mice (Janvier, France) were housed in a temperature (19.5-24.5°C), relative humidity (40-70%) and 12-12h light-dark cycle (light 7:00 a.m. to 7:00 p.m.)-controlled room, with ad *libitum* access to filtered (0.22 µm) tap-water and irradiated pelleted laboratory chow (ref. A04, U.A.R., France) throughout the study. They were housed 5 per cage and a 21-day acclimatization period was observed.

The mice were treated during 14 consecutive days (T1 to T14), with morning and afternoon gavages with the various treatments as described below.

At T15, the mice were weighed and blood samples collected without anticoagulant by retro-orbital puncture under CO₂/O₂ anesthesia.

Triglycerides were measured at T0 and T15 using the multi-parametric analyzer.
Veh/Veh = Vehicle in morning/Vehicle in the afternoon
A300/Veh = metformin, 300 mg/kg in the morning/ Vehicle in the afternoon
B100/B100 = fenofibrate, 100 mg/kg in the morning/fenofibrate, 100 mg in the afternoon
A300B100/B100 = metformin, 300 mg/kg in the morning, and fenofibrate, 100 mg/kg in the morning/fenofibrate, 100 mg/kg in the afternoon
A100/Veh = metformin, 100 mg/kg in the morning/Vehicle in the afternoon
B30/B30 = fenofibrate, 30 mg/kg in the morning/fenofibrate, 30 mg/kg in the afternoon
A100B30/B30 = metformin, 100 mg/kg in the morning, and fenofibrate, 30 mg/kg in the afternoon/fenofibrate, 30 mg/kg in the afternoon

The results are summarized in Table 2.

**Table 2**

| Triglycérides (g/l) | | | |
|---|---|---|---|
| | T0 | T15 | % var. T0/T15 |
| *Veh*/*Veh* (n=10) | 2.01 ± 0.13 | 2.12 ± 0.11 | 9.5 |
| *A300*/*Veh* (n=10) | 1.81 ± 0.14 | 1.67 ± 0.06* | -3,3° |
| *B100*/*B100* (n=9) | 1.89 ± 0.13 | 0.85 ± 0.08* | -53,5* |
| *A300B100*/*B100* (n=7) | 1.76 ± 0.11 | 0.56 ± 0.02* | -66.8* |
| *A100*/*Veh* (n=10) | 1.68 ± 0.12 | 1.79 ± 0.16° | 7.3° |
| *B30*/*B30* (n=8) | 1.72 ± 0.12 | 0.97 ± 0.08* | -42,3* |
| *A100B30*/*B30* (n=9) | 1.91 ± 0.15 | 1.01 ± 0.09* | -48.3* |

| | | | |
|---|---|---|---|
| Values are expressed as mean ± SEM *Statistics* One-way analysis of variance followed by a Dunnett's test. ° non significant as compared to vehicle | | | |
| *p<0.01 | | | |

These data demonstrate the following:
- As far as metformin treatment is concerned, no reduction in triglycerides level was seen.
- As far as fenofibrate treatment is concerned, the triglycerides level was significantly decreased.
- As far as the co-administration of fenofibrate and metformin is concerned, a further decrease of the triglycerides level was observed.

## Claims

1. Use of a PPARα agonist, metformin and a pharmaceutically acceptable carrier for the manufacture of a pharmaceutical formulation for decreasing serum triglycerides.

2. Use of a PPARα agonist, metformin and a pharmaceutically acceptable carrier for the manufacture of a pharmaceutical formulation for the treatment of metabolic syndrome.

3. Use of a PPARα agonist, metformin and a pharmaceutically acceptable carrier for the manufacture of a pharmaceutical formulation for the treatment of obesity.

4. The use according to one of claim 1 to 3, wherein the PPARα agonist is a fibrate selected from the group consisting of gemfibrozil, fenofibrate, bezafibrate, clofibrate, ciprofibrate.

5. The use according to claim 4, wherein the fibrate is fenofibrate.

6. The use according to one of claims 1 to 5, wherein the effective dosage of the PPARα agonist is in the range of about 10 to about 3000 mg per day.

7. The use according to one of claims 1 to 6, wherein the effective dosage of metformin is in the range of about 10 to about 3000 mg per day.

8. The use according to one of claims 1 to 7, wherein the PPARα agonist and metformin are administered simultaneously.

9. The use according to one of claims 1 to 7, wherein the PPARα agonist and metformin are administered sequentially.

10. Use of a PPARα agonist and metformin for the manufacture of a kit for decreasing serum triglycerides, for the treatment of metabolic syndrome or for the treatment of obesity, the kit comprising two separate compositions, the first comprising the PPARα agonist and the second comprising metformin or a pharmaceutically acceptable salt thereof.
